# EUROPÄISCHE PATENTSCHRIFT

(11) **EP 1 543 789 B1**
(45) Veröffentlichungstag und Bekanntmachung des Hinweises auf die Patenterteilung: **04.10.2006**
(21) Anmeldenummer: 04010353.3
(22) Anmeldetag: 30.04.2004
(51) Int. Cl.: A61B 19/00

(54) **Universelles Instrument bzw. Instrumentensatz zur Navigation in der computergestützten Chirurgie**
Universal instrument or set of instruments for navigation in computer-aided surgery
Instrument universel ou ensemble d' instruments pour la navigation en chirurgie assistée par ordinateur

(30) Priorität: 17.12.2003 DE 10359296
(43) Veröffentlichungstag der Anmeldung: 22.06.2005
(73) Patentinhaber: BrainLAB AG, 85551 Kirchheim/Heimstetten (DE)
(72) Erfinder: Lechner, Christian, 82287 Jesenwang (DE); Schaffrath, Claus, Dr., 81377 München (DE)
(74) Vertreter: Schwabe - Sandmair - Marx

(56) Entgegenhaltungen:
- DE-U- 20 203 439
- US-A1- 2002 035 321
- US-B1- 6 556 857

## Beschreibung

Die Erfindung betrifft ein universelles Instrument bzw. einen Instrumentensatz für die Computer gestützte Chirurgie.

Zur Navigation von unterschiedlichen Instrumenten für die Wirbelsäulenchirurgie (wie z.B. Ahle, Pfriem, Meißel, etc.) werden solche Instrumente derzeit für die Computer gestützte Chirurgie (CGC) an der Wirbelsäule intra-operativ kalibriert. Dazu wird ein Instrumenten-Referenzstem auf das entsprechende Instrument geklemmt und dieses anschließend unter Zuhilfenahme eines speziellen Kalibrierblocks (ICM - Instrument Calibration Matrix) kalibriert. Alles in allem ist dieser Kalibrierungsschritt sehr zeitaufwändig und unkomfortabel für den Anwender. Darüber hinaus besteht das Risiko, dass sich die Instrumenten-Referenzsteme nach der Kalibrierung, sprich während der Anwendung, lockern können und die Kalibrierung und damit die Genauigkeit verfälschen. Zudem verbirgt die Kalibrierung an sich mögliche Fehlerquellen für spätere Navigations-Ungenauigkeiten. Wenn der Anwender diese Ungenauigkeiten der Instrumentenkalibrierung nicht bemerkt, könnte dies zu Verletzungen des Patienten führen.

Bei der CGC an der Wirbelsäule wird die Navigation hauptsächlich dazu verwendet, Implantatschrauben akkurat in die Pedikel des Wirbelkörpers zu platzieren, ohne das Rückenmark oder Spiralnerven oder andere vital-notwendigen Organe oder Blutgefäße zu verletzen. Die spinalen Instrumente dienen hierbei zur Präparierung und Eröffnung des Pedikels für die anschließend korrekte Einbringung der Pedikelschrauben.

Vorkalibrierte Instrumente sind meist für die CGC erforderlich, um die mühsame und je nach Anwender fehleranfällige Kalibrierung zu umgehen und eine höhere Genauigkeit in die Navigation der Instrumente zu erreichen.

Es ist bereits ein drehbarer Instrumentenadapter für die spinale CGC aus der US 6,021,343 bekannt. Diese Lösung umfasst einen Zwischenstückadapter mit drehbar gelagertem Referenzstern, der vom Navigationssystem erkannt wird. Der Adapter hat an seinen beiden Enden jeweils einen Schnellverschluss. Auf der einen Seite können unterschiedliche Instrumentenspitzen hineingesteckt werden, wobei an der anderen Seite ebenfalls unterschiedliche Griffe oder Bohrmaschinen adaptiert werden können. Der Zwischenstückadapter kann dann zur Navigation unterschiedlicher spinaler Instrumente verwendet werden, die jedoch, um korrekt dargestellt werden zu können, rotationssymmetrisch sein müssen.

In der US 6,556,857 wird erwähnt, dass ein rotierbarer Zwischenstückadapter einen Arretiermechanismus in einer von der Software bekannten Stellung besitzen kann. Allerdings werden die Ausführungsform und die Funktionsweise eines solchen Arretiermechanismus für die Navigation nicht beschrieben.

Es ist die Aufgabe der vorliegenden Erfindung, ein Instrument für den Einsatz in der Computer gestützte Chirurgie bzw. einen Instrumentensatz zur Verfügung zu stellen, mit denen die Nachteile des Standes der Technik überwunden werden. Insbesondere soll eine hohe Genauigkeit in der Anbringung des Referenzsterns relativ zum Instrument erzielt werden, und damit auch eine hohe Genauigkeit bei der Software-Erkennung des Referenzsterns und damit auch der Navigation des Instruments. Eine weitere Aufgabe besteht darin, auch nicht-rotationssymmetrische Instrumente vorkalibriert in die Computer gestützte Chirurgie implementieren zu können.

Diese Aufgabe wird gemäß der vorliegenden Erfindung durch ein Instrument gemäß dem Patentanspruch 1 gelöst. Ein erfindungsgemäßer Instrumentensatz gemäß dem Patentanspruch 13 gestattet eine universelle Anwendbarkeit des Erfindungsprinzips.

Gemäß der vorliegenden Erfindung ist der Referenzstem-Adapter direkt am Instrumentenschaft um diesen drehbar angeordnet. Damit hebt sich die vorliegende Erfindung von dem bekannten "Zwischenstückadapter"-Lösungen ab. Hierdurch ist eine größere Genauigkeit erzielbar, da eine zusätzliche Schnittstelle zwischen Instrumentenspitze und Referenzstem-Adapter, und damit eine zusätzliche Fehlerquelle ausgeschlossen wird.

Besonders die Winkelgenauigkeit der Position des Referenzsterns relativ zur Instrumentenachse wird entscheidend verbessert.

Die Unteransprüche beschreiben bevorzugte Ausführungsformen der vorliegenden Erfindung. Die hierin beschriebenen Merkmale können einzeln oder in jedweder Kombination verwirklicht werden.

Gemäß einer Ausführungsform der vorliegenden Erfindung ist der Referenzstern-Adapter an einer Instrumentenschaft-Adaptionsschnittstelle angeordnet. Dabei besteht die Möglichkeit, dass die Instrumentenschaft-Adaptionsschnittstelle und der sie umgebende Teil des Referenzstern-Adapters eine lösbare Drehwinkelarretierung aufweisen. Diese Drehwinkelarretierung ist bevorzugt so auszuführen, dass der Referenzstern bei der Arbeit immer den gleichen Winkel am Instrumentenschaft einhält, jedoch auch noch immer die Möglichkeit besteht, diesen Winkel zu verändern. Dazu kann die Drehwinkelarretierung eine überwindbare Reibungs- oder Formkopplung umfassen, insbesondere ein Kugeldruckstück am Umgriffsteil des Referenzstern-Adapters, dass in über den Umfang verteilte Längskerben der Instrumentschaft-Adaptionsschnittstelle eingreifen kann.

Ein erfindungsgemäßes Instrument kann so ausgebildet sein, dass die Adaptionsschnittstelle eine Referenz-Axialanschlagfläche für den Referenzstern-Adapter aufweist. Damit wird eine vorbestimmbare Axialposition für den Referenzstern-Adapter bereitgestellt.

Vorteilhafterweise ist an einer Schnittstelle zwischen Instrumentenschaft-Adaptionsschnittstelle und Referenzstern-Adapter mindestens ein Referenzstem-Orientierungsmittel angeordnet. Dieses Referenzstern-Orientierungsmittel kann eine oder mehrere besondere Winkelpositionen für den Referenzstern-Adapter am Instrumentenschaft festlegen, und vorteilhafterweise schafft ein solches Referenzstern-Orientierungsmittel eine einstellbare, insbesondere lösbar arretierbare Positionierung zwischen Instrumentenschaft-Adaptionsschnittstelle und Referenzstern-Adapter (Winkelpositionierung), wobei gemäß einer bevorzugten Ausführung ein Orientierungs-Pin an der Referenz-Axialanschlagfläche und mindestens ein Orientierungsschlitz am Umgriffsteil des Referenzstern-Adapters bereitgestellt werden.

In vorteilhafter Ausführung der vorliegenden Erfindung ist an dem Instrumentenschaft, insbesondere an oder einstückig mit der Instrumentenschaft-Adaptionsschnittstelle eine Griffstückverbindung vorgesehen. An dieser kann ein Griffstück angebracht sein, insbesondere mit einem verdrehsicheren Schnellverschluss, wobei das Griffstück die Axialbewegung des Referenzstern-Adapters limitiert.

Ein erfindungsgemäß ausgebildetes Instrument ist mit Vorteil so ausgestaltet, dass der Referenzstern-Adapter und der Instrumentenschaft, insbesondere die Instrumentenschaft-Adaptionsschnittstelle, Ausrichtungsmittel aufweisen, die verhindern, dass der Referenzstem-Adapter verkehrt herum auf den Instrumentenschaft aufgesetzt wird.

Dies kann durch besondere Eingriffe dieser beiden Teile an einer Seite ermöglicht werden, insbesondere auch über die schon vorher benannten Orientierungs-Pins und Orientierungsschlitze.

Gemäß einer weiteren bevorzugten Ausführungsform weist der Instrumentenschaft zur eindeutigen softwaremäßigen Erkennung mindestens einen instrumentenspezifisch angeordneten Markierungspunkt auf, insbesondere eine Einkerbung zur Abtastung durch einen Pointer eines chirurgischen Navigationssystems.

Instrumentenschaft und/oder Instrumentenspitze können bei einem erfindungsgemäßen Instrument entweder rotationssymmetrisch oder nicht-rotationssymmetrisch ausgestaltet sein. Die oben genannte Ausführungsform mit den Orientierungsmitteln, also insbesondere mit den Orientierungs-Pins und den Orientierungsschlitzen eignet sich speziell für nicht-rotationssymmetrische Instrumente.

Bei einem erfindungsgemäßen Instrumentsatz weisen bevorzugt mindestens beide Instrumente des Satzes einheitliche Griffstückverbindungen für unterschiedliche Griffstücke mit einheitlichen Schnittstellen an den Griffstücken auf. Ferner besteht die Möglichkeit, dem Instrumentenschaft unterschiedliche Instrumente zur eindeutigen softwaremäßigen Erkennung instrumentenspezifisch unterschiedlich angeordnete Markierungspunkte zu geben, insbesondere die schon vorher genannten Einkerbungen zur Abtastung durch einen Pointer eines chirurgischen Navigationssystems. Jedes Instrument des erfindungsgemäßen Instrumentensatzes kann ein spinales Instrument sein, wie z.B. eine Ahle, ein Meißen oder ein Pfriem, und der Instrumentensatz kann solche unterschiedlichen chirurgischen Instrumente umfassen. Natürlich ist die Erfindung aber auch bei sonstigen chirurgischen Instrumenten anwendbar, ob rotationssymmetrisch oder nicht-rotationssymmetrisch, beispielsweise im Bereich der Trauma- und Orthopädiebehandlung (Hüfte, Knie usw.) mit Hilfe der chirurgischen Navigation bzw. der Computer gestützten Chirurgie.

Die Erfindung wird im Weiteren anhand von Ausführungformen und unter Bezugnahme auf die beiliegenden Zeichnungen näher erläutert. Es zeigen:
- Figur 1: einen Satz unterschiedlicher spinaler Instrumente, wie z.B. Ahle und Pfriem, die gemäß der vorliegenden Erfindung ausgestaltet sind;
- Figur 2: ein erfindungsgemäß ausgestaltetes chirurgisches Instrument in einer Längs-Schnittansicht;
- Figur 3: eine Vergrößerung der Ansicht aus Figur 2 im Bereich der Anbringung eines Referenzsterns;
- Figur 4: eine obere Ansicht auf das Instrument der Figur 2;
- Figur 5: eine perspektivische Gesamtansicht sowie eine vergrößerte Ausschnittsansicht, die das Anbringen eines Referenzstern-Adapters an einem Instrumentenschaft zeigt;
- Figur 6: einen Screenshot einer Software zur Auswahl vorkalibrierter Instrumente; und
- Figur 7: eine perspektivische Darstellung sowie eine Ausschnittsvergrößerung zur Software-Erkennung erfindungsgemäßer Instrumente mittels einer Schaftmarkierung.

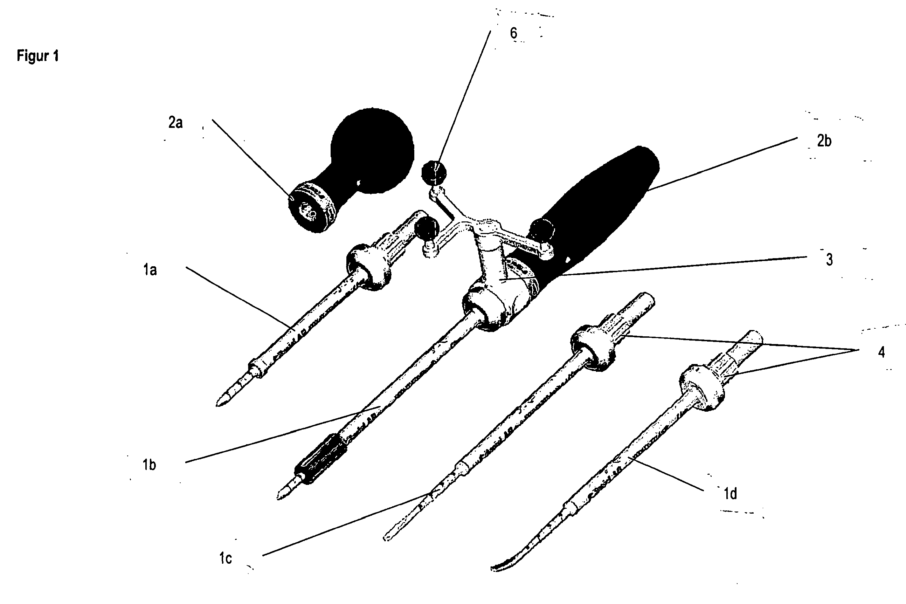

Der in Figur 1 dargestellte Instrumentensatz verdeutlicht, wie die vorliegende Erfindung universell auf verschiedene Instrumente angewendet werden kann, die in der Computer gestützten Chirurgie verwendet werden. Es sind unterschiedliche Instrumentenschäfte 1a bis 1d dargestellt, wie z.B. Ahle und Pfriem. Die Instrumente bzw. deren Schäfte können je nach geforderter Anwendung unterschiedliche Form und Länge haben und sie müssen nicht unbedingt rotationssymmetrisch sein, wie an den Instrumentenschäften 1c und 1d zu erkennen ist.

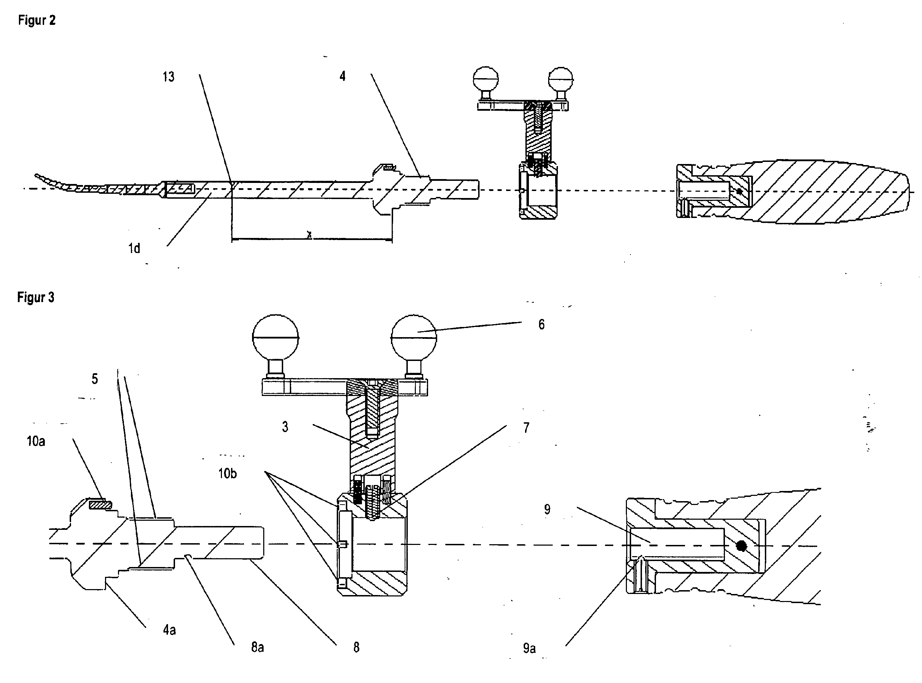

Jeder Instrumentenschaft 1a bis 1d hat aber am proximalen Ende eine einheitliche Adaptionsschnittstelle 4, die genauer in den Explosions-Schnittansichten der Figuren 2 und 3 dargestellt ist, und die im Folgenden noch eingehender beschrieben wird.

Des Weiteren sind in der Figur 1 noch Beispiele für unterschiedliche Griffstücke 2a, 2b gezeigt, sowie ein Referenzstern-Adapter 3, der mit passiven Markern 6 versehen werden kann.

Unter Bezugnahme auf die Figuren 1, 2 und 3 wird nunmehr näher erläutert, wie die einzelnen Instrumententeile erfindungsgemäß zum Zusammenbau angepasst sind. Der Referenzstem-Adapter 3 wird gemäß der vorliegenden Erfindung direkt und drehbar am Instrumentenschaft angeordnet, und zwar auf der Adaptionsschnittstelle 4, die in einem erfindungsgemäßen Instrumentensatz unterschiedlicher Instrumente einheitlich ausgestaltet ist. Die Adaptionsschnittstelle 4 weist bei der dargestellten Ausführungsform an ihrer zylindrischen Oberfläche Rastkerben 5 auf, die in konstantem Abstand über den Umfang verteilt sind, z.B. 12 Rastkerben im Abstand von 30°. Andere Rasteinrichtungen sind möglich. Ferner umfasst die Adaptionsschnittstelle 4 noch eine Referenzfläche 4a, die einen axialen Anschlag für den Referenzstern-Adapter 3 bildet.

Beim Zusammenbau des erfindungsgemäß ausgestalteten Instruments wird der drehbare Referenzstern-Adapter 3 direkt auf der Adaptionsschnittstelle 4 des Instrumentenschaftes 1a bis 1d platziert. Eine korrekte axiale Platzierung ist nur von einer Seite aus möglich, so dass der Referenzstern-Adapter 4 nicht versehentlich um 180° verdreht montiert werden kann. Der Referenzstern-Adapter 4 hat passive Marker 6, die für die Erkennung des Instruments durch ein chirurgisches Navigationssystem, insbesondere durch die Kameras des Systems benötigt werden.

Zu seinem inneren axialen Durchgang hin weist der Referenzstern-Adapter 3 ein zentrisches Kugeldruckstück auf, das in eine der Rastkerben 5 einrasten kann und so verhindert, dass sich der Referenzstern-Adapter 3 unbeabsichtigt verdreht, also beispielsweise aus dem Sichtfeld der Kameras wegdreht, wenn er während der Anwendung gerade nicht von Hand gehalten wird.

Dadurch, dass der Referenzstem-Adapter 3 erfindungsgemäß direkt auf den Instrumentenschaft, das heißt auf der Adaptionsschnittstelle 4 platziert wird, können höhere Genauigkeiten der navigierten Instrumentenachse erreicht werden, und zwar insbesondere im Vergleich zu denjenigen Instrumenten, wo zusätzliche Schnittstellen vorgesehen werden. Das axiale Spiel wird durch die Griffstücke 2a, 2b limitiert, die mittels eines Schnellverschlusses auf das proximale Ende des Instrumentenschaftes gesteckt werden können. Zu diesem Zweck hat jeder Instrumentenschaft eine Schnittstelle 8 (Figur 3) für die Anbringung unterschiedlicher Griffe, die beispielsweise in der Figur 1 mit den Bezugszeichen 2a und 2b dargestellt sind. Unterschiedliche Griffe bedeutet hier die Ausgestaltung des Hand-Griffstückes selbst, die Schnittstelle 8 und das Gegenstück 9 im Griff sind bei allen Griffen einheitlich ausgestaltet.

Die Griffe 2a, 2b werden in der Art eines Schnellverschlusses mit dem Instrumentenschaft verbunden. Bei der dargestellten bevorzugten Ausführungsform haben sie einen Passungs-Schlitz 9 mit einem seitlich in diesen einragenden Kugeldruckstück 9a. Das Griffstück wird mit dem Passungs-Schlitz 9 auf die proximale Schnittstelle 8 am Instrumentenschaft gesteckt, bis das Kugeldruckstück 9a in eine entsprechende Kerbe 8a einrastet. Damit wird die Stellung des Griffstücks auf der Schnittstelle 8 axial festgelegt und limitiert auch die Bewegungsfreiheit des dazwischen liegenden Referenzstern-Adapters 3 in axialer Richtung. Der ansonsten runde Instrumentenschaft im Bereich der Schnittstelle 8 kann an einer Seite, beispielsweise an der Seite der Kerbe 8a abgeflacht sein, wobei der Passungs-Schlitz 9 dann dieselbe Abflachung aufweist. Dadurch wird verhindert, dass sich der Griff durchdreht, wenn Drehmomente vom Griff auf den Instrumentenschaft übertragen werden. Grundsätzlich ist auch die Verwendung eines anderes Schnellverschluss-Prinzips möglich.

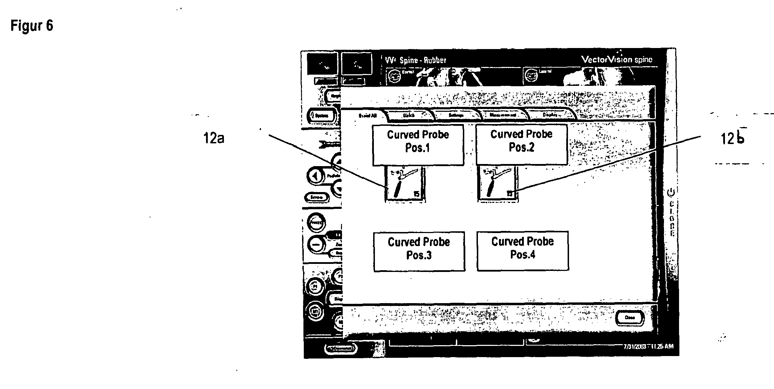

Falls das Instrument ein nicht-rotationssymmetrisches Instrument ist, wie dies beispielsweise bei dem Instrumentenschäften 1c und 1d (Figur 1) der Fall ist, kann erfindungsgemäß auch die Orientierung des Instruments in immer derselben Richtung sichergestellt werden. Dies geschieht gemäß der dargestellten bevorzugten Ausführungsform dadurch, dass im Bereich der Referenzfläche 4a an einer spezifischen Stelle (die beispielsweise dem Winkel nach definiert ist) ein Orientierungs-Pin 10a vorgesehen ist. Das Orientierungs-Hilfsmittel, also z.B. der Orientierungs-Pin 10a ist je nach Instrumentenform an einer speziellen Stelle angeordnet, also beispielsweise an einer speziellen Winkelposition. Dies gilt auch für das passende Gegenstück am Referenzstern-Adapter 3, wo auch mehrere solcher Gegenstücke angeordnet sein können. Wie speziell aus den Figuren 3 und 5 hervorgeht, hat der Referenzstern-Adapter 3 bei der dargestellten Ausführungsform vier Orientierungsschlitze 10b, so dass er in vier vorgegebenen Stellungen, von denen zwei in der vergrößerten Darstellung in der Figur 5 näher erkennbar und mit 11a und 11b bezeichnet sind, am nicht-rotationssymmetrischen Instrument arretiert werden kann, wobei der Orientierungs-Pin 10a in den entsprechenden Orientierungsschlitz 10b eingreift. Der Anwender kann die Position auswählen und einstellen, so dass die Referenzstern-Orientierung im Verhältnis zum Instrument die beste Sichtbarkeit für die Navigationskameras während der Anwendung gewährleistet. Die Orientierung des nicht-rotationssymmetrischen Instruments relativ zum Referenzstern-Adapter 3 ist für jede Stellung, z.B. 11a, 11b in der Software implementiert, die für die Computer gestützte Chirurgie verwendet wird. Der Anwender kann dann, wie in Figur 6 mittels eines Screenshots dargestellt ist, die eingestellte Instrumentenposition (z.B. 11a (Pos. 1), 11b (Pos. 2)) in der Software in einer speziellen Auswahlseite für vorkalibrierte Instrumente auswählen. Auf dem Screenshot der Figur 6 sind diese Auswahltasten mit den Bezugszeichen 12a und 12b angezeigt. Hierdurch wird sichergestellt, dass das nicht-rotationssymmetrische Instrument korrekt von der Navigationssoftware dargestellt wird.

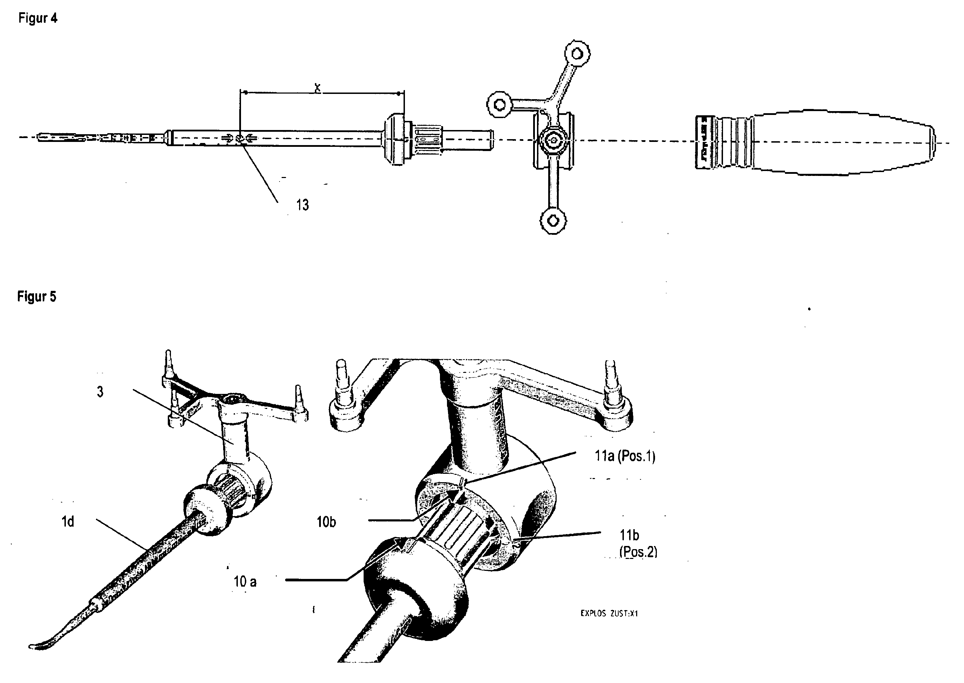

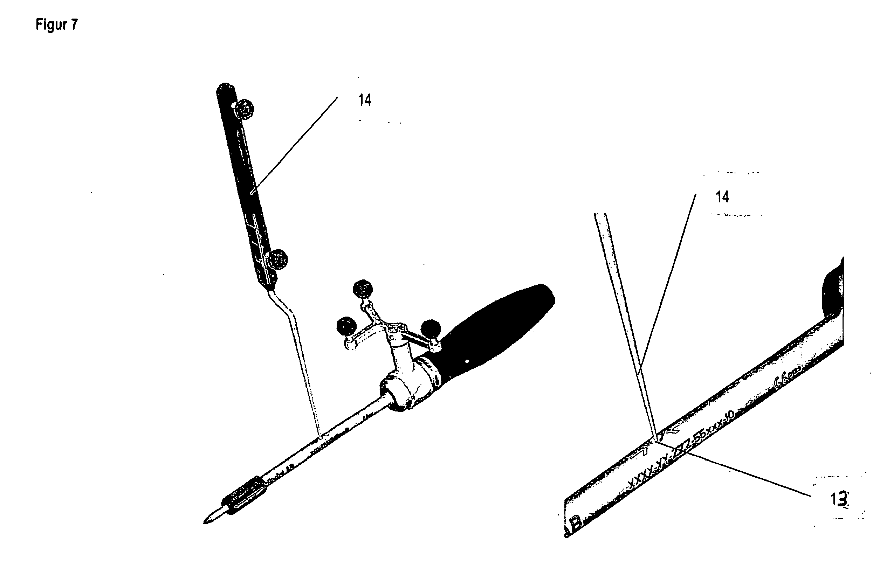

Unter Bezugnahme auf die Figuren 2, 4 und 7 wird nunmehr näher auf die softwaregestützte Instrumentenauswahl und genaue Instrumentenverifikation bei der Verwendung erfindungsgemäßer Instrument bzw. Instrumentensätze eingegangen. Jedes Instrument bzw. jeder Instrumentenschaft 1a bis 1d weist einen Markierungspunkt auf, der bei den dargestellten Ausführungsformen eine Einkerbung 13 ist. Die Markierung bzw. Einkerbung 13 ist bei jedem Instrument individuell an einer anderen Seite angebracht und hat einen instrumentenspezifischen Abstand X (Figur 4) in Bezug zum Referenzstern bzw. in Bezug zur Referenzfläche 4a an der Adaptionsschnittstelle 4. Diese Markierung bzw. Einkerbung 13 kann mit einem Pointer 14 abgegriffen werden, der beispielsweise in Figur 7 dargestellt ist und ebenfalls vom Navigationssystem positionell erfasst werden kann. Aus dem bekannten Abstand der Pointerspitze 14 am Markierungspunkt 13 zum Referenzstern kann dann von der Software das entsprechende Instrument erkannt und sofort richtig dargestellt werden, ohne es softwaremäßig auswählen zu müssen. Dies hat auch den Vorteil, dass kein Instrument versehentlich falsch ausgewählt werden kann oder fälschlich dargestellt wird, da immer das real vorhandene Instrument korrekt verifiziert wird.

Des Weiteren kann der Markierungspunkt bzw. die Einkerbung 13 auch zur Genauigkeits-Verifizierung vor jeder Anwendung für das vorkalibrierte Instrument verwendet werden. Der Pointer 14 wird dann mit seiner Spitze wieder am Markierungspunkt 13 platziert, und seine von der Navigationssoftware dargestellte Position am Instrument wird mit der realen Position verglichen. Nur wenn der Pointer auch korrekt von der Navigationssoftware mit der Spitze am Markierungspunkt 13 dargestellt wird, ist die Genauigkeit hinreichend gut und das Instrument kann verwendet werden.

Obwohl die vorliegende Erfindung anhand von Ausführungsbeispielen für spinale Instrumente erörtert wurde, ist sie nicht auf solche Instrumente beschränkt, sondern kann auch auf Instrumente der Trauma- und Orthopädie (z.B. Hüfte/Knie) -Navitationsapplikationen angewendet werden. Dementsprechend kann ein erfindungsgemäßer Instrumentensatz mit neuen applikationsspezifischen Instrumenten erweitert werden.

## Patentansprüche

1. Instrument für den Einsatz in der Computer gestützten Chirurgie mit einem Instrumentenschaft (1) und mit einem daran befestigten Referenzstern-Adapter (3) **dadurch gekennzeichnet, dass** der Referenzstern-Adapter (3) direkt am Instrumentenschaft (1) um diesen drehbar an einer Instrumentenschaft-Adaptionsschnittstelle (4) angeordnet ist, welche eine vorbestimmbare Axial position für den Referenzstern-Adapter (3) bereitstellt.

2. Instrument nach Anspruch 1, **dadurch gekennzeichnet, dass** die Instrumentenschaft-Adaptionsschnittstelle (4) und der sie umgebende Teil des Referenzstern-Adapters (3) eine lösbare Drehwinkelarretierung aufweisen.

3. Instrument nach Anspruch 2, **dadurch gekennzeichnet, dass** die Drehwinkelarretierung eine überwindbare Reibungs- oder Formkopplung umfasst, insbesondere ein Kugeldruckstück (7) am Umgriffsteil der Referenzstern-Adapters (3), das in über den Umfang verteilte Längskerben (5) der Instrumentenschaft-Adaptionsschnittstelle (4) eingreifen kann.

4. Instrument nach einem der Ansprüche 1 bis 3, **dadurch gekennzeichnet, dass** die Adaptionsschnittstelle (4) eine Referenz-Axialanschlagfläche (4a) für den Referenzstern-Adapter (3) aufweist.

5. Instrument nach einem der Ansprüche 1 bis 4, **dadurch gekennzeichnet, dass** an einer Schnittstelle zwischen Instrumentenschaft-Adaptionsschnittstelle (4) und Referenzstern-Adapter (3) mindestens ein Referenzstern-Orientierungsmittel angeordnet ist.

6. Instrument nach Anspruch 5, **dadurch gekennzeichnet, dass** ein Referenzstem-Orientierungsmittel eine einstellbare, insbesondere lösbar arretierbare Positionierung zwischen Instrumentenschaft-Adaptionsschnittstelle (4) und Referenzstern-Adapter (3) schafft, insbesondere mittels eines Orientierungs-Pins (10a) an der Referenz-Axialanschlagfläche (4a) und mindestens einem Orientierungsschlitz (11a, 11b) am Umgriffsteil des Referenzstern-Adapters (3).

7. Instrument nach einem der Ansprüche 1 bis 6, **dadurch gekennzeichnet, dass** an dem Instrumentenschaft, insbesondere anschließend an oder einstückig mit der Instrumentenschaft-Adaptionsschnittstelle (4) eine Griffstückverbindung vorgesehen ist.

8. Instrument nach Anspruch 7, **dadurch gekennzeichnet, dass** an der Griffstückverbindung (8) ein Griffstück (2a, 2b) angebracht ist, insbesondere mit einem verdrehsicheren Schnellverschluss, wobei das Griffstück (2a, 2b) die Axialbewegung des Referenzstern-Adapters (3) limitiert.

9. Instrument nach einem der Ansprüche 1 bis 8, **dadurch gekennzeichnet, dass** der Referenzstern-Adapter (3) und der Instrumentenschaft, insbesondere die Instrumentenschaft-Adaptionsschnittstelle (4), Ausrichtungsmittel - aufweisen, die verhindern, dass der Referenzstern-Adapter (3) verkehrt herum auf den Instrumentenschaft aufgesetzt wird.

10. Instrument nach einem der Ansprüche 1 bis 9, **dadurch gekennzeichnet, dass** der Instrumentenschaft (1) zur eindeutigen softwaremäßigen Erkennung mindestens einen instrumentenspezifisch angeordneten Markierungspunkt (13) aufweist, insbesondere eine Einkerbung zur Abtastung durch einen Pointer eines chirurgischen Navigationssystems.

11. Instrument nach einem der Ansprüche 1 bis 10, **dadurch gekennzeichnet, dass** es einen rotationssymmetrischen Instrumentenschaft und/oder eine rotationssymmetrische Instrumentenspitze aufweist.

12. Instrument nach einem der Ansprüche 1 bis 10, **dadurch gekennzeichnet, dass** es einen nicht-rotationssymmetrischen Instrumentenschaft und/oder eine nichtrotationssymmetrische Instrumentenspitze aufweist.

13. Instrumentensatz mit mindestens zwei Instrumenten nach einem der vorhergehenden Ansprüche, **dadurch gekennzeichnet, dass** mindestens zwei der Instrumente des Satzes einheitliche Instrumentenschaft-Adaptionsschnittstellen (4) aufweisen.

14. Instrumentensatz nach Anspruch 13, **dadurch gekennzeichnet, dass** mindestens zwei der Instrumente des Satzes einheitliche Griffstückverbindungen für unterschiedliche Griffstücke mit einheitlichen Schnittstellen an den Griffstücken aufweisen.

15. instrumentensatz nach Anspruch 13 oder 14, **dadurch gekennzeichnet, dass** der Instrumentenschaft (1) unterschiedlicher Instrumente zur eindeutigen softwaremäßigen Erkennung instrumentenspezifisch unterschiedlich angeordnete Markierungspunkte (13) aufweist, insbesondere Einkerbungen zur Abtastung durch einen Pointer eines chirurgischen Navigationssystems.

16. Instrumentensatz nach einem der Ansprüche 13 bis 15, **dadurch gekennzeichnet, dass** der unterschiedliche chirurgische Instrumente wie Ahle, Meißel oder Pfriem umfasst.

## Claims

1. An instrument for use in computer-aided surgery, comprising an instrument shaft (1) and a reference star adaptor (3) fixed to it, **characterised in that** the reference star adaptor (3) is directly arranged on the instrument shaft (1), rotatable about it, on an instrument shaft adapting interface (4) which provides a predetermined axial position for the reference star adaptor (3).

2. The instrument according to claim 1, **characterised in that** the instrument shaft adapting interface (4) and the part of the reference star adaptor (3) surrounding it comprise a detachable angular rotational catch.

3. The instrument according to claim 2, **characterised in that** the angular rotational catch comprises a surmountable frictional or positive coupling, in particular a ball-thrust piece (7) on the wrap-around part of the reference star adaptor (3), which can engage with circumferentially distributed, longitudinal notches (5) of the instrument shaft adapting interface (4).

4. The instrument according to any one of claims 1 to 3, **characterised in that** the adapting interface (4) comprises a reference axial stopper area (4a) for the reference star adaptor (3).

5. The instrument according to any one of claims 1 to 4, **characterised in that** at least one reference star orientating means is arranged at an interface between the instrument shaft adapting interface (4) and the reference star adaptor (3).

6. The instrument according to claim 5, **characterised in that** a reference star orientating means creates a settable, in particular detachably catching positioning between the instrument shaft adapting interface (4) and the reference star adaptor (3), in particular by means of an orientating pin (10a) on the reference axial stopper area (4a), and at least one orientating slit (11a, 11b) on the wrap-around part of the reference star adaptor (3).

7. The instrument according to any one of claims 1 to 6, **characterised in that** a grip piece connection is provided on the instrument shaft, in particular connected to or integral with the instrument shaft adapting interface (4).

8. The instrument according to claim 7, **characterised in that** a grip piece (2a, 2b) is attached to the grip piece connection (8), in particular via a non-rotational quick-release lock, wherein the grip piece (2a, 2b) limits the axial movement of the reference star adaptor (3).

9. The instrument according to any one of claims 1 to 8, **characterised in that** the reference star adaptor (3) and the instrument shaft, in particular the instrument shaft adapting interface (4), comprise aligning means which prevent the reference star adaptor (3) from being placed onto the instrument shaft the wrong way round.

10. The instrument according to any one of claims 1 to 9, **characterised in that** in order to be clearly recognised via the software, the instrument shaft (1) comprises at least one marking point (13) arranged instrument-specifically, in particular an indentation to be traced by a pointer of a surgical navigation system.

11. The instrument according to any one of claims 1 to 10, **characterised in that** it comprises a rotationally symmetrical instrument shaft and/or a rotationally symmetrical instrument tip.

12. The instrument according to any one of claims 1 to 10, **characterised in that** it comprises a rotationally asymmetrical instrument shaft and/or a rotationally asymmetrical instrument tip.

13. A set of instruments comprising at least two instruments according to any one of the preceding claims, **characterised in that** at least two of the instruments of the set comprise uniform instrument shaft adapting interfaces (4).

14. The set of instruments according to claim 13, **characterised in that** that at least two of the instruments of the set comprise uniform grip piece connections for different grip pieces with uniform interfaces on the grip pieces.

15. The set of instruments according to claim 13 or 14, **characterised in that** in order to be clearly recognised via the software, the instrument shaft (1) of different instruments comprises marking points (13) which are differently arranged instrument-specifically, in particular indentations to be traced by a pointer of a surgical navigation system.

16. The set of instruments according to any one of claims 13 to 15, **characterised in that** it includes different surgical instruments such as an awl, chisel or bodkin.

## Revendications

1. Instrument destiné à être utilisé en chirurgie assistée par ordinateur, comportant une tige d'instrument (1) et un raccord d'étoile de référence (3), fixé sur celle-ci, **caractérisé en ce que** le raccord d'étoile de référence (3) est disposé directement sur la tige d'instrument (1) de manière à pouvoir tourner autour de celle-ci au niveau d'une interface d'adaptation (4) de la tige d'instrument qui fournit une position axiale pouvant être prédéterminée pour le raccord d'étoile de référence (3) .

2. Instrument selon la revendication 1, **caractérisé en ce que** l'interface d'adaptation (4) de la tige d'instrument et la partie du raccord d'étoile de référence (3), qui entoure ladite interface, comportent un dispositif d'arrêt d'angle de rotation libérable.

3. Instrument selon la revendication 2, **caractérisé en ce que** le dispositif d'arrêt d'angle de rotation comporte un couplage par frottement ou emboîtement surmontable, en particulier un élément d'appui à bille (7) sur la partie de prise de l'étoile de référence (3), lequel élément peut pénétrer dans des rainures longitudinales (5) réparties sur le pourtour de l'interface d'adaptation (4) de la tige d'instrument.

4. Instrument selon l'une quelconque des revendications 1 à 3, **caractérisé en ce que** l'interface d'adaptation (4) comporte une surface de butée axiale de référence (4a) pour l'étoile de référence (3).

5. Instrument selon l'une quelconque des revendications 1 à 4, **caractérisé en ce qu'**au moins un moyen d'orientation de l'étoile de référence est disposé sur une interface entre l'interface d'adaptation (4) de tige d'instrument et l'étoile de référence (3).

6. Instrument selon la revendication 5, **caractérisé en ce qu'**un moyen d'orientation de l'étoile de référence assure un positionnement réglable, en particulier blocable de manière libérable, entre l'interface d'adaptation (4) de tige d'instrument et l'étoile de référence (3), en particulier au moyen d'un ergot d'orientation (10a) sur la surface de butée axiale de référence (4a) et au moins une fente d'orientation (11a, 11b) sur la partie de prise de l'étoile de référence (3).

7. Instrument selon l'une quelconque des revendications 1 à 6, **caractérisé en ce que** sur la tige d'instrument est prévu une connexion de manche, en particulier adjacente à l'interface d'adaptation (4) de tige d'instrument ou d'un seul tenant avec cette dernière.

8. Instrument selon la revendication 7, **caractérisé en ce qu'**un manche (2a, 2b) est monté sur la connexion de manche (8), en particulier avec un raccord rapide bloqué en rotation, le manche (2a, 2b) limitant le mouvement axial du raccord d'étoile de référence (3).

9. Instrument selon l'une quelconque des revendications 1 à 8, **caractérisé en ce que** le raccord d'étoile de référence (3) et la tige d'instrument, en particulier l'interface d'adaptation (4) de la tige d'instrument, comportent des moyens d'alignement qui empêchent que le raccord d'étoile de référence (3) soit mis en place à l'envers sur la tige d'instrument.

10. Instrument selon l'une quelconque des revendications 1 à 9, **caractérisé en ce que**, pour une reconnaissance logicielle univoque, la tige d'instrument (1) comporte au moins un point de repère, situé de manière spécifique à l'instrument, en particulier une encoche destinée à être palpée par un pointeur d'un système de navigation chirurgical.

11. Instrument selon l'une quelconque des revendications 1 à 10, **caractérisé en ce qu'**il comporte une tige d'instrument à symétrie de révolution et/ou une pointe d'instrument à symétrie de révolution.

12. Instrument selon l'une quelconque des revendications 1 à 10, **caractérisé en ce qu'**il comporte une tige d'instrument sans symétrie de révolution et/ou une pointe d'instrument sans symétrie de révolution.

13. Ensemble d'instruments comportant au moins deux instruments selon l'une quelconque des revendications précédentes, **caractérisé en ce qu'**au moins deux des instruments de l'ensemble comportent des interfaces d'adaptation d'instruments (4) uniformes.

14. Ensemble d'instruments selon la revendication 13, **caractérisé en ce qu'**au moins deux des instruments de l'ensemble comportent des connexions de manches uniformes pour différents manches avec des interfaces uniformes sur les manches.

15. Ensemble d'instruments selon la revendication 13 ou 14, **caractérisé en ce que**, pour une reconnaissance logicielle univoque, la tige d'instrument (1) de différents instruments comporte des points de repère (13) agencés différemment de manière spécifique à l'instrument, en particulier des encoches destinées à être palpées par un pointeur d'un système de navigation chirurgical.

16. Ensemble d'instruments selon l'une quelconque des revendications 13 à 15, **caractérisé en ce qu'**il comporte différents instruments de chirurgie, tels qu'une alène, un trépan ou un poinçon.
